# EUROPEAN PATENT SPECIFICATION

(11) **EP 4 106 729 B1**
(45) Date of publication and mention of the grant of the patent: **14.08.2024**
(21) Application number: 21705518.5
(22) Date of filing: 16.02.2021
(51) Int. Cl.: A61K 9/107, A61K 39/00, A61K 39/39, A61K 47/69, A61K 49/18, A61P 29/00, A61P 37/02, A61P 37/06

(54) **AMPHIPHILIC POLYMERS AND THEIR USE FOR IMPROVED PRODUCTION OF NANOPARTICLES FOR THE TARGETED DELIVERY OF ANTIGENS**
AMPHIPHILE POLYMERE UND DEREN VERWENDUNG ZUR VERBESSERTEN HERSTELLUNG VON NANOPARTIKELN ZUR GEZIELTEN ABGABE VON ANTIGENEN
POLYMÈRES AMPHIPHILES ET LEUR UTILISATION POUR AMÉLIORER LA PRODUCTION DE NANOPARTICULES POUR L'ADMINISTRATION CIBLÉE D'ANTIGÈNES

(30) Priority: 17.02.2020 EP 20157797
(43) Date of publication of application: 28.12.2022
(73) Proprietor: Topas Therapeutics GmbH, 20251 Hamburg (DE)
(72) Inventor: DIGIGOW, Reinaldo, 20251 Hamburg (DE); MUNGALPARA, Disha, 20251 Hamburg (DE); POHLNER, Johannes, 20251 Hamburg (DE); SELECI, Muharrem, 20251 Hamburg (DE)
(74) Representative: Uexküll & Stolberg
(86) International application number: PCT/EP2021/053711
(87) International publication number: WO 2021/165227

(56) References cited:
- EP-A1- 2 591 801
- WO-A1-2017/136652
- US-A1- 2018 028 647
- KELLER S. ET AL.: "Neutral polymer micelle carriers with pH-responsive, endosome-releasing activity modulate antigen trafficking to enhance CD8+ T cell responses", JOURNAL OF CONTROLLED RELEASE, vol. 191, 2014, pages 24 - 33, XP009521815

## Description

### FIELD OF THE INVENTION

The present invention provides nanoparticles for use in the prevention and treatment of autoimmune diseases, allergies or other chronic inflammatory conditions, and for generation of regulatory T cells. In particular, the present invention relates to nanoparticles comprising a micelle comprising an amphiphilic polymer with a number average molecular weight (Mn) of 6,000 to 1,000 g/mol, rendering the nanoparticle water-soluble, and at least one peptide comprising at least one T cell epitope. The present invention also relates to a pharmaceutical composition comprising the nanoparticles. The pharmaceutical composition can be used for generating regulatory T cells specific to at least one T cell epitope in a subject for treating or preventing a disease wherein suppression of a specific immune response is beneficial.

Furthermore, the present invention provides a method for the production of the nanoparticles.

### BACKGROUND OF THE INVENTION

Autoimmune diseases represent a substantial burden for patients and healthcare systems. Current therapies rely mostly on immunosuppressive drugs with considerable side effects. Autoantigen-specific immunotherapies that exclusively target disease-specific immune pathologies leaving the general immune status untouched represent an unmet medical need.

Immune tolerance to self-antigens is maintained by multiple mechanisms that control potentially pathogenic autoreactive lymphocytes, including deletion, clonal anergy or suppression by regulatory T cells. Autoimmune disease may thus result from insufficient control of autoreactive lymphocytes, and a major goal of immunotherapy for autoimmune diseases is the induction of tolerance to autoantigens by restoring regulation. A particularly promising way to restore self-tolerance seems to be the manipulation of autoantigen-specific CD4+CD25+FOXP3+ regulatory T cells. The adoptive transfer of these cells can prevent autoimmune or inflammatory conditions.

The liver plays a central role in the suppression of unwanted immune responses against blood-borne antigens, *e.g.* food antigens, entering the circulation. This fundamental mechanism of the liver can be employed to specifically downregulate detrimental immune responses against external protein antigens or autoantigens. Antigenic peptides derived from such proteins, when coupled to nano-sized carriers and administered intravenously, mimic food antigens triggering uptake by specific liver cells, the liver sinusoidal endothelial cells (LSECs), followed by a tolerogenic immune response. Peptide-specific immune tolerance can thus be induced for defined immune disease-causing antigens, leading to the amelioration or even eradication of detrimental immune reactions. This approach can thus be used to treat ongoing diseases and may also be used to prevent the respective diseases in a preventive setting.

For example, ectopic expression of a neuroantigen in the liver can prevent autoimmune neuroinflammation in mice with experimental autoimmune encephalomyelitis (EAE), an animal model for multiple sclerosis (MS). This finding can be explained by the capacity of the liver to generate neuroantigen-specific regulatory T cells (Tregs) that have a profound ability to control and suppress autoimmune responses. LSECs play a crucial role for achieving this effect. LSECs express MHC/HLA class I and class II molecules on their surface and thus have the capacity to present peptides to both, CD8+ (via cross-presentation) and CD4+ T cells, respectively. Peptide-antigen presentation by LSECs converts naive and T effector cells to Tregs in an antigen-specific way *in vitro.* This is apparently the physiological mechanism by which LSECs can establish tolerance against blood-borne antigens.

Nanoparticles conjugated with a disease-specific antigenic peptide to the particle surface, like blood-borne antigens, target the liver after intravenous injection. Upon uptake by LSECs, presumably by pinocytosis, the nanoparticles accumulate in the endosomal compartment where the peptide antigens are released from the surface of the particles. This leads to the presentation of those antigenic peptides at the LSEC surface, mediated by MHC/HLA molecules. There is evidence that the subsequent generation of Tregs confers immune tolerance specific for the respective autoantigen based on its antigenic peptide epitopes.

WO 2009/067349 discloses a pharmaceutical composition comprising a biocompatible nanoparticle linked to an aryl hydrocarbon receptor (AHR) transcription factor ligand for use in the treatment of autoimmune disorders by increasing the number and/or activity of regulatory T cells.

WO 2013/072051 discloses a pharmaceutical composition for use in generating regulatory T cells specific to at least one T cell epitope in a subject for treating or preventing a disease wherein suppression of a specific immune response is beneficial. The nanoparticle comprises a micelle comprising an amphiphilic polymer rendering the nanoparticle water-soluble, and a peptide comprising at least one T cell epitope associated with the outside of the micelle. It is generally suggested to use commercially available poly(maleic anhydride-alt-1-octadecene as amphiphilic polymer having a molecular mass of 30,000 to 50,000 g/mol and about 90% purity.

However, in certain medical applications it is important that the nanoparticles can be produced with a very high degree of purity using efficient purification methods.

There still is a need in the art for improved nanoparticles for treating and preventing a disease wherein suppression of a specific immune response is beneficial, e.g. in autoimmune diseases, in allergies, in transplantation, in the suppression of anti-drug-antibodies (ADA) against therapeutics or gene vectors, or in a disease wherein inflammation is excessive, chronic or adverse, and wherein said pharmaceutical composition is suitable for use in human subjects.

### SUMMARY OF THE INVENTION

According to the present invention the above problems are solved by a nanoparticle comprising
a) a micelle comprising an amphiphilic polymer with a number average molecular weight (Mn) of 6,000 to 1,000 g/mol, and
b) at least one peptide comprising at least one T cell epitope.

The inventors have surprisingly found that nanoparticles comprising an amphiphilic polymer with a number average molecular weight (Mn) of 6,000 to 1,000 g/mol can be produced more easily and to a higher degree of purity.

Without being bound to a theory underlying the effects of the nanoparticles of the present invention, it is presently understood that upon uptake of the nanoparticles by LSECs, the at least one peptide associated with the outside of the micelle is released, either by hydrolysis, proteolysis or other activities in the endosomes, processed as if it was a blood-born antigen, and presented to T cells in a tolerogenic environment. The low molecular weight amphiphilic polymer enables excretion of individual polymer molecules upon release *in vivo.* This provides for hepatobiliary excretion, which appears to represent the preferred excretion pathway for nanoparticles.

It has been surprisingly found that low molecular weight amphiphilic polymers having a number average molecular weight (Mn) of 6,000 to 1,000 g/mol can easily be excreted. It is also expected that low molecular weight amphiphilic polymers and their metabolites are rapidly eliminated from the body after treatment.

The inventors have surprisingly found that the low molecular weight amphiphilic polymer has advantageous properties during production of the nanoparticles of the present invention. The low molecular weight amphiphilic polymer produces fewer aggregates during coating of a solid core than a high molecular weight amphiphilic polymer. In addition, the low molecular weight amphiphilic polymer can be purified more efficiently compared to a high molecular weight amphiphilic polymer. In particular, unbound polymer can be separated more efficiently when a low molecular weight amphiphilic polymer is used in the nanoparticle of the invention.

The invention further provides a pharmaceutical composition comprising the nanoparticle.

The present invention also provides a pharmaceutical composition comprising the nanoparticle for use in generating regulatory T cells specific to at least one T cell epitope in a subject for treating or preventing a disease wherein suppression of a specific immune response is beneficial.

Finally, the present invention provides a method of producing a nanoparticle comprising:
i) obtaining an amphiphilic polymer with a number average molecular weight (Mn) of 6,000 to 1,000 g/mol,
ii) optionally purifying the amphiphilic polymer,
iii) forming micelles of the amphiphilic polymer,
iv) adding at least one peptide to form the nanoparticles.

### BRIEF DESCRIPTION OF THE FIGURES

Figure 1 is a flow chart showing the synthesis of an iron oleate complex.
Figure 2 is a flow chart showing the synthesis of superparamagnetic iron oxide nanoparticles (SPIONs).
Figure 3 is a flow chart showing the synthesis of low molecular weight poly (maleic anhydride-*alt*-1-octadecene) (LM-PMAOD).
Figure 4 is a flow chart showing the synthesis of low molecular weight poly(maleic acid-*alt*-1-octadecene) (LM-PMAcOD).
Figure 5 is a flow chart showing the polymer coating of SPIONs.
Figure 6 is a flow chart showing the coupling of peptides to the nanoparticles of the present invention.
Figure 7 shows transmission electron microscopy (TEM) images of nanoparticles of the present invention (100x magnification).
Figure 8 is a graph representing the molecular mass distribution of LM-PMAcOD as determined using gel permeation chromatography and polystyrene as a calibration standard.
Figures 9 to 11 illustrate SEC chromatograms of the purification of HM-PMAcOD-SPION samples.
Figure 12 shows the results of size exclusion chromatography of the purification LM-PMAcOD-SPION samples.
Figure 13 shows SEC chromatograms of the products after coating.

### DETAILED DESCRIPTION OF THE INVENTION

According to the present invention nanoparticles are provided which comprise a micelle comprising an amphiphilic polymer with a number average molecular weight (Mn) of 6,000 to 1,000 g/mol rendering the nanoparticle water-soluble, and at least one peptide comprising at least one T cell epitope. The nanoparticles may further comprise a solid hydrophobic core which is coated by the micelle.

According to the present application, the term "nanoparticle" is used interchangeably with "nanoscale particle". Such particles have a diameter of 1 to 999 nm, preferably, of 2 to 600 nm, 5 to 500 nm, 10 to 300 nm, 30 to 100 nm or 40 to 50 nm.

In the context of the present invention, a nanoparticle is a structure formed by at least a micelle and a peptide which is associated to the micelle. The peptides may either be associated to the outside of the micelle or encapsulated inside the micelle.

In an embodiment of the present invention, the nanoparticles of the present invention comprise a solid hydrophobic core, a micelle coating the core comprising an amphiphilic polymer with a number average molecular weight (Mn) of 6,000 to 1,000 g/mol rendering the nanoparticle water-soluble, and at least one peptide comprising at least one T cell epitope.

### The micelle

In the context of the present invention, the term "micelle" relates to an aggregate of amphiphilic molecules dispersed in an aqueous solution. The hydrophilic parts of the amphiphilic molecules are in contact with the surrounding solvent, sequestering the hydrophobic "tail" regions of the amphiphilic molecules on the inside of the micelle, and thus render the nanoparticle water-soluble. This type of micelle is also known as a normal phase micelle (or oil-in-water micelle).

The micelle can be formed by one, but also by more than one, *e.g.,* two, three or four amphiphilic polymeric molecules. The micelle can be formed by the same or by different amphiphilic polymeric molecules. In general, in the context of the specification, "a" or "the" is not intended to be limiting to "one" unless specifically stated.

In a preferred embodiment, the micelle is formed by a single layer of amphiphilic polymers.

Such a micelle can be structurally distinct from a bilayer or a liposome formed by an amphiphilic polymer. In this case the structures are not, or not to a significant percentage (e.g. not more than 10%, more than 5%, or preferably, more than 1%), comprised in the nanoparticle of the present invention.

In one embodiment of the present invention, the amphiphilic polymer is used to produce at least 70%, preferably at least 90% of the micelle. In a preferred embodiment, the micelle consists of the amphiphilic polymer.

In some embodiments of the present invention the nanoparticles do not comprise a solid hydrophobic core. In other embodiments, the nanoparticles comprise the micelle and a solid hydrophobic core.

Methods of producing the nanoparticles of the present invention are described in detail bellow.

### The amphiphilic polymer

The amphiphilic polymer of the present invention generally comprises a hydrophobic region comprising a hydrophobic aliphatic chain having a length of 8 to 23, preferably 8 to 21, most preferably 16 to 18 carbon atoms.

The hydrophilic region of the amphiphilic polymer may be negatively charged in an aqueous solution.

In a preferred embodiment of the present invention, the amphiphilic polymer spontaneously forms micelles in solution. When a solid hydrophobic core is present, the amphiphilic polymer forms micelles around the solid core, rendering the nanoparticle water-soluble.

The number average molecular weight (Mn) of the amphiphilic polymer is 1,000 to 6,000 g/mol.

The number average molecular weight may be determined using gel permeation chromatography (GPC), preferably using polystyrene as calibration standard.

In a preferred embodiment the number average molecular weight is determined using a PL-gel mixed D column at a temperature of 40°C, a mobile phase consisting of tetrahydrofuran/acetic acid 90/10% (v/v), a flow rate of 1.0 ml/min, in combination with a refractive index detector at a temperature of 35°C and polystyrene as calibration standard.

In the most preferred embodiment, the determination of the number average molecular weight uses GPC and the following measurement conditions:

| Reference standards | Polystyrene standard (MW (nominal Mp); 1000 g/mol to 130000 g/mol |
|---|---|
| Column | Agilent PL-gel mixed-D, 300 x 7.5 mm ID, 5 µm |
| Column Temperature | 40°C |
| Detector | Refractive index detector at 35°C |
| Flow rate | 1.0 ml/min |
| Injection volume | 20 µL |
| Autosampler temperature | Ambient |
| Run time | 15 min |
| Mobile phase | Tetrahydrofuran/Acetic acid [90/10]%(v/v) |
| Mobile phase program | Isocratic |

The amphiphilic polymer may be an alternating copolymer. An alternating copolymer is a copolymer comprising two species of monomeric units distributed in alternating sequence.

In one embodiment of the present invention, the amphiphilic polymer is a copolymer of maleic anhydride and at least one alkene.

The alkene used in the production of the amphiphilic polymer may be selected from one or more of 1-decene, 1-undecene, 1-dodecene, 1-tridecene, 1-tetradecene, 1-pentadecene, 1-hexadecene, 1-heptadecene, 1-octadecene, 1-nonadecene or 1-eicosene, preferably the alkene is 1-octadecene.

In a preferred embodiment of the present invention, the amphiphilic polymer is a copolymer of maleic anhydride and an alkene.

In a preferred embodiment of the present invention, the amphiphilic polymer has a main hydrophilic poly-maleic anhydride backbone having hydrophobic alkyl side chains. Typically, the side chain can have from 5 to 23 carbon atoms, in particular from 9 to 21 atoms. In a most preferred embodiment, the side chains are linear and have from 10 to 18 carbon atoms.

The amphiphilic polymer may comprise the following building block 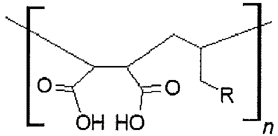 wherein R is a hydrocarbyl group or a substituted hydrocarbyl group. In a preferred embodiment of the present invention, R is a C₄ to C₂₂ alkyl group, such as a C₇ to C₁₉ alkyl group.

In an even more preferred embodiment, R is a linear alkyl group, preferably a linear C₇ to C₁₇ alkyl group, most preferably R is a linear pentadecyl group or a linear nonyl group.

The amphiphilic polymer may consist of the building block defined above.

In other embodiments according to the present invention, the amphiphilic polymer comprises at least 50 %, preferably at least 70 %, most preferably more than 90 % of the building block defined above.

In a preferred embodiment, the amphiphilic polymer is selected from the group comprising poly(maleic acid-1-octadecene), poly(maleic acid-1-tetradecene) or poly(maleic acid-1-dodecene), preferably the polymer is poly(maleic acid-1-octadecene) and the number average molecular weight of the polymer is from 6,000 to 1,000 g/mol.

In a specifically preferred embodiment, the amphiphilic polymer is selected from the group comprising poly(maleic acid-*alt*-1-octadecene), poly(maleic acid-alt-1-dodecene) and poly(maleic acid-*alt*-1-tetradecene), preferably the polymer is poly(maleic acid-*alt*-1-octadecene) and the number average molecular weight of the polymer is from 5000 to 1000 g/mol.

Methods of producing the amphiphilic polymer of the present invention are also described in detail below.

### The peptides

The nanoparticle of the present invention further comprises at least one peptide comprising at least one T cell epitope. The peptide may be associated with the outside of the micelle or encapsulated in the inside of the micelle (in embodiments where no solid hydrophobic core is present in the nanoparticle of the present invention). Accordingly, the peptide may be localized on the outside of the micelle or inside the micelle.

The peptide may be covalently linked to the micelle or non-covalently associated, preferably covalently linked to the micelle.

In a preferred embodiment, the peptide is covalently linked to the micelle using a method of covalently coupling peptides known in the art such as carbodiimide or succinimide coupling. Preferably, the peptide is covalently linked to the micelles using 1-Ethyl-3-(3-dimethylaminopropyl)carbodiimide (EDC) chemistry.

In the context of the present invention, the term "peptide" is not intended to be limiting in size, in particular, the peptide may comprise a whole protein or 8 to 2,000 amino acids, preferably, 8 to 200 amino acids, 8 to 100 amino acids, 9 to 60 amino acids, or 10 to 20 amino acids. The term also comprises combinations of different peptides, which may be linked to each other as fusion polypeptides.

In a preferred embodiment of the present invention, the peptide comprises 10 to 20, such as 13 to 17 amino acids.

In a specifically preferred embodiment, the peptide comprises 15 amino acids.

The peptide comprises at least one T cell epitope. Methods for identifying T cell epitopes and selected T cell epitopes are well known in the art and described for example in the publications of Rammensee et al., 1999 (Immunogenetics 50 213-219) and Sanchez-Trincado et al. 2017 (J Immunol Res. 2017:2680160. doi: 10.1155/2017/2680160. Epub 2017 Dec 28). In the context of the present invention a T cell epitope is a peptide sequence inducing regulatory T cells. At least one epitope needs to be capable of being presented by cells of the subject to which the nanoparticles are to be administrated. Preferably, the peptide comprises several epitopes which enable it to be presented in a plurality of Major Histocompatibility Complex types.

As the regulatory T cells are predominantly CD4+, presentation on MHC class II is of main interest. The HLA type of this subject, *e.g*., a human subject, and can easily be tested as part of the selection of epitopes. Epitopes of a specific peptide which can be presented on specific MHC molecules are known and/or can routinely be selected, *e.g.,* by appropriate software.

Peptides are designed based on published data to make sure that they - in association with the specified HLA restriction element - bind MHC/HLA with high affinity and profoundly stimulate and activate T cells. Ideally, peptides of choice are inferred from naturally processed peptides and characterized as immunodominant.

The peptide may be synthesized, recombinantly expressed or isolated or modified from natural sources. The peptide, or at least the epitope against which regulatory T cells are to be generated, is preferably derived from a peptide/protein against which an inflammatory immune response is to be suppressed, *e.g.,* in the context of treatment or prevention of an autoimmune disease or an allergy. The peptide may, *e.g.,* be an allergen, a known autoimmune antigen, or a fragment or derivative thereof. The peptide can combine various epitopes from various antigens.

In a preferred embodiment of the present invention, the peptide is an antigenic peptide derived from Desmoglein-3 (Dsg3). For example, the peptide may be one or more than one of the Desmoglein-3 peptides characterized by SEQ ID NOs:1-3.

According to one embodiment of the present invention, the nanoparticles comprise only one type of peptide comprising at least one T cell epitope.

According to a further embodiment the present invention provides a composition comprising different nanoparticles, wherein each nanoparticle comprises numerous peptides having the same amino acid sequence but the composition comprises mixtures of nanoparticles which differ from each other in the peptide sequence. The composition may for example comprise different nanoparticles comprising 2 to 6 different peptides. In one aspect, the composition of different nanoparticles may comprise three different types of nanoparticles, each characterized by one of SEQ ID NOs:1-3.

### The solid hydrophobic core

In one embodiment of the present invention the nanoparticle comprises a solid hydrophobic core at least partially coated by the micelle.

The core can be an inorganic core, preferably comprising iron oxide, CdSe, silver or gold.

The diameter of the core may be 2 to 500 nm, preferably, 3 to 25 nm, more preferably, 5 to 15 nm. The diameter of the core may be determined using transmission electron microscopy (TEM) or small-angle X-ray scattering (SAXS).

Exemplary inorganic cores are iron oxide nanoparticles stabilized by oleic acid or another carboxylic acid (C₁₄-C₂₂, preferably, C₁₆-C₁₈), quantum dots (CdSe/CdS/ZnS stabilized, *e.g.,* by trioctyloxinphosphinoxide), gold nanoparticles, *e.g*., stabilized by sulfonic compounds.

Such inorganic cores by themselves are typically not stable in an aqueous solvent such as water, but embedding them in the polymeric micelles renders them water-soluble. The hydrophobic parts of the amphiphilic polymer interact with the hydrophobic core of the nanoparticle, leading to formation of a single coating layer of polymer surrounding the core. In the coating process the amphiphilic polymer can replace the hydrophobic part of the core by ligand exchange and the double layer micelle is thus formed around the core. In one embodiment of the invention, the polymer at least partially replaces the oleic acid on the surface of the core particle and the hydrophilic part of the polymer interacts with the surface of the iron oxide core and the hydrophobic part of the polymer interact with each other forming a double layer micelle around the iron oxide core, resulting in an iron oxide coated with polymer.

According to a preferred embodiment of the present invention, the core is superparamagnetic.

In a specifically preferred embodiment of the present invention, the core is a superparamagnetic iron oxide nanoparticle (SPION), which may be stabilized by oleic acid.

The cores preferably render the nanoparticles of the invention traceable, *e.g*., by their characteristics in fluorescence, electron microscopy or other detection method.

### The nanoparticles

The inventors have found that nanoparticles for use in the present invention are suitable for transferring the peptide to liver sinusoidal endothelial cells of a subject *in vivo.*

The nanoparticles may additionally comprise a moiety, *e.g*., a carbohydrate or a protein targeting them, or enhancing targeting to specific cells such as liver sinusoidal endothelial cells and/or Kupffer cells. Such moiety could, e.g., enhance or accelerate uptake from the circulation via receptor mediated endocytosis. Examples of suitable modifications are carbohydrates such as mannose.

The nanoparticle, by virtue of the polymer forming the micelle, may be negatively charged or uncharged; preferably, the nanoparticle is negatively charged at a pH of 6 to 7. The polymer coating may comprise acid, *e.g.,* carboxylic acid, groups, leading to a negative charge of the nanoparticle.

The nanoparticles of the present invention may have a zeta potential between -20 and -50 mV, preferably between -25 and -45 mV, more preferably between -28 and -42 mV at pH 6 to 7 (pH during measurement). The zeta potential can be measured using a Malvern Zetasizer Nano ZS instrument.

The nanoparticles of the present invention may have a hydrodynamic diameter (z-average) between 10 and 100 nm or 10 and 70, preferably between 10 and 50, more preferably between 20 and 40 m, most preferably between 22 and 32 nm, as measured by dynamic light scattering (DLS).

The nanoparticles of the present invention may have a polydispersity index below 0.50, preferably between 0.05 and 0.45, more preferably between 0.10 and 0.40, as measured by dynamic light scattering (DLS).

The determination of the hydrodynamic diameter and the polydispersity index is carried out using electrophoretic light scattering analysis methods, preferably a Malvern Zetasizer. In one embodiment the method for determining the hydrodynamic diameter and the polydispersity index is carried out using electrophoretic light scattering, disposable polystyrene cuvettes, Zetasizer Software 7.12, milli-Q water. The nanosphere size standards of 20 nm and 100 nm (NIST certified or equivalent) are diluted in an aqueous 0.9% sodium chloride solution and the test samples are diluted in water. All aqueous reagents are filtered through 0.22 um membrane prior to use. In the most preferred embodiment of the invention, the method for determining the hydrodynamic diameter and the polydispersity index is carried out using electrophoretic light scattering in combination with the following analysis conditions:

### Overview of the analysis conditions:

| **Parameter** | **Setting** |
|---|---|
| Dispersant name | Water |
| Dispersant RI | 1.33 |
| Viscosity (cP at 25,0 °C) | 0.8872 |
| Material RI (sample) | 2.42 |
| Material RI (standards) | 1.333 |
| Material Absorption | 0.05 |
| Temperature (°C) | 25 |
| Measurement Position (mm) | 4.65 |
| Cell description | Disposable sizing cuvette |
| Attenuator | Auto |
| Measurement duration | Auto |

The evaluation of the data is based on mean diameter (Z-Average, nm by intensity), which is a parameter also known in DLS as the cumulants mean and Polydispersity index (PDI), which is used as a measure of the size distribution.

Furthermore, the nanoparticles of the present invention may have a total polymer content of 0.1 to 5 mg/mL, preferably 0.5 to 4 mg/mL, more preferably of 1 to 3 mg/mL. The total polymer content is determined by GPC. For measuring the total polymer content, the peptides are hydrolyzed, and the particles are destroyed (e.g using a 6 M HCl solution). The polymer is extracted after addition of EDTA. After evaporation of solvent, the residue is re-dissolved and the polymer content is determined by GPC.

The determination of total polymer content is preferably carried out using the following reagents and reference standards water (HPLC grade), acetonitrile (HPLC grade), tetrahydrofuran with BHT (THF-HPLC grade), acetic acid 100° (analytical grade), hydrochloric acid 37% (analytical grade), ethylenediaminetetraacetic acid disodium salt dihydrate (analytical grade), ethyl acetate (analytical grade), sodium hydroxide (analytical grade) and poly(maleic acid-alt-1-octadecene) as reference material.

The chromatographic conditions for the determination of total polymer content are:

| Column | Agilent PL-gel Mixed-D, 300+75 mm ID, 5µm |
|---|---|
| Column temperature | 40°C |
| Flow rate | 1.0 mL/min |
| Detector | Refractive index detector at 35°C |
| Sample rating | 2.31 Hz or equivalent |
| Injection volume | 20 µL |
| Autosampler temperature | Ambient |
| Run time | 15 min |
| Mobile phase | THF/Acetic acid [90/10]%(v/v) |
| Mobile phase program | Isocratic |

In a specifically preferred embodiment of the present invention, the nanoparticles comprise an iron oxide core encapsulated by a coating of poly(maleic acid-*alt*-1-octadecene) having a number average molecular weight from 6,000 to 1,000 g/mol and a peptide comprising at least one T cell epitope peptide which is preferably covalently linked to the micelle.

In one aspect the present invention thus provides nanoparticles comprising
a) a micelle comprising an amphiphilic polymer comprising the following building block 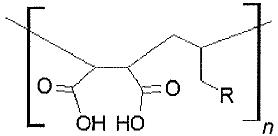 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) at least one peptide comprising at least one T cell epitope; and
c) a solid hydrophobic core which is at least partically coated by the micelle, wherein the core comprises a traceable inorganic material selected from the group comprising iron oxide, CdSe/CdS/ZnS, silver and gold.

The number average molecular weight of the polymer is preferably from 6,000 to 1,000 g/mol.

The nanoparticle preferably has a hydrodynamic diameter between 50 and 10 nm as measured by dynamic light scattering.

### The pharmaceutical composition

The invention further provides a pharmaceutical composition comprising nanoparticles of the present invention.

The peptides used may be present in the pharmaceutical composition in a concentration from 0.01 to 2 mM, preferably from 0.1 to 1 mM, most preferably 0.45 mM to 1 mM.

In a preferred embodiment, the amount of free (unbound) polymer in the composition is less than 10%, preferable less than 5%, most preferable less than 2% of the total amount of polymer.

The pharmaceutical composition of the present invention may further comprise at least one suitable excipient and/or diluent. The diluent is preferably water or water-based, *e.g*., a buffer such as Phosphate buffered saline (PBS), Ringer solution, TRIS buffer or sodium chloride solution. Suitable preservatives may or may not be contained.

It is evident that, in particular for administration to a human subject, the composition preferably is sterile and biologically compatible.

In a preferred embodiment of the present invention, the pharmaceutical composition comprises the nanoparticles of the present invention dispersed in D-mannitol, TRIS and/or L-lactic acid.

In an embodiment of the present invention, the pharmaceutical composition comprises nanoparticles of the present invention which do not comprise a solid hydrophobic core, which nanoparticles are dispersed in D-mannitol, TRIS and/or L-lactic acid. The use of this buffer has the advantage that the particles are very stable in this buffer and can be lyophilized later on.

Furthermore, the pharmaceutical composition may comprise more than one type of nanoparticle of the present invention, wherein the different types of nanoparticles have different peptides associated with the outside of the micelle. By using a mixture of nanoparticles, broader immune tolerance can be induced by several autoantigenic peptides at the same time. These peptides may be derived from a single immunogenic protein, or from different proteins.

In a preferred embodiment of the present invention, the pharmaceutical composition comprises between 2 and 6 different types of nanoparticles, wherein preferably all associated peptides of the different types of nanoparticles comprise at least one T cell epitope.

In a specifically preferred embodiment of the present invention, the pharmaceutical composition comprises between 3 and 4 different types of nanoparticles, wherein all associated peptides of the different types of nanoparticles comprise at least one T cell epitope. In particular, each nanoparticle may be associated with a different antigenic peptide derived from Dsg3 .

The pharmaceutical composition may comprise the nanoparticle in a concentration below 100 µM, preferably from 0.5 to 80 µM, most preferably from 1 to 50 µM. If more than one nanoparticle is present in the pharmaceutical composition, each may be present in a concentration below 100 µM, preferably from 0.5 to 80 µM, more preferably from 1 to 50 µM.

The pharmaceutical composition of the present invention may comprise different types of nanoparticles in equimolar concentration.

In one aspect the present invention thus provides a pharmaceutical composition comprising nanoparticles comprising
a) a micelle comprising an amphiphilic polymer comprising the following building block 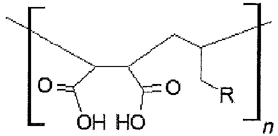 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) at least one peptide comprising at least one T cell epitope; and
c) a solid hydrophobic core which is at least partically coated by the micelle, wherein the core comprises a traceable inorganic material selected from the group comprising iron oxide, CdSe/CdS/ZnS, silver and gold.

The number average molecular weight of the polymer in the pharmaceutical composition will preferably range from 6,000 to 1,000 g/mol.

The nanoparticles in the pharmaceutical composition of the present invention preferably have a hydrodynamic diameter between 50 and 10 nm as measured by dynamic light scattering.

In a preferred aspect the present invention provides a pharmaceutical composition comprising at least three different types of nanoparticles, wherein each nanoparticle comprises
a) a micelle comprising an amphiphilic polymer comprising the following building block 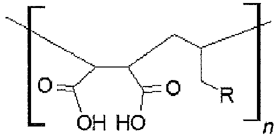 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) one peptide comprising at a T cell epitope; and
c) a solid hydrophobic core which is at least partically coated by the micelle, wherein the core comprises a traceable inorganic material selected from the group comprising iron oxide, CdSe/CdS/ZnS, silver and gold; and
wherein the three different types of nanoparticles differ among each other in the peptide sequence, wherein the first type of nanoparticles comprises peptides having SEQ ID NO:1, the second type of nanoparticles comprises peptides having SEQ ID NO:2 and the third type of nanoparticles comprises peptides having SEQ ID NO:3.

### The medical use

The pharmaceutical composition of the present invention is intended for use in and formulated for administration to a subject having a disease wherein suppression of a specific immune response is beneficial.

The pharmaceutical compositions may be administered to a subject in need thereof.

The required dose and concentration for administration to the subject may be determined by the responsible medical attendant according to the facts and circumstances of the case. An exemplary dose might comprise 0.03 pmol to 0.90 pmol per patient body weight, *e.g*., for a human subject.

Administration may be repeated, e.g., twice, three or four times, *e.g.,* with, 1, 2, 3, 4, 5, 6, 7, 10 or 14 days between administrations.

Preferably, the pharmaceutical composition is for use in suppressing a specific immune response, such as treating or preventing a disease wherein suppression of a specific immune response is beneficial. More preferably, the pharmaceutical composition is for use in generating regulatory T cells specific to at least one T cell epitope in a subject for treating or preventing a disease wherein suppression of a specific immune response is beneficial.

The disease can be an autoimmune disease associated with defined autoantigens. In the context of the present invention the term "autoimmune disease" is understood defined by Hayter et. al. (Autoimmunity Reviews 11 (2012) 754-765). In a preferred embodiment, the autoimmune disease is selected from the group comprising Pemphigus vulgaris, Pemphigus foliaceus, Epidermolysis bullosa Acquisita, Bullous pemphigoid, Cicatricial pemphigoid, Goodpasture syndrome, Microscopic polyangiitis, Granulomatosis with polyangiitis (Granulom. Wegener), Thrombotic thrombocytopenic purpura, Immune thrombocytopenic purpura, Uveitis, HLA-B27-associated acute anterior uveitis, Multiple sclerosis, Neuromyelitis optica, Type I diabetes, Narcolepsy with or without cataplexy, Celiac disease, Dermatitis herpetiformis, Allergic airways disease/Asthma, Myasthenia gravis, Hashimoto thyreoiditis, Autoimmune thyroid disease, Graves disease, Autoimmune thyroid disease, Autoimmune Hypoparathyroidism, Autoimmune thyroid disease, Antiphospholipid syndrome, Autoimmune Addison's Disease, Autoimmune haemolytic anaemia, Chronic inflammatory demyelinating, Polyneuropathy, Guillain-Barré syndrome, Autoimmune neutropenia, Linear morphea, Batten disease, Acquired hemophilia A, Relapsing polychondritis, Isaac's syndrome (acquired neuro-myotonia), Rasmussen encephalitis, Morvan syndrome, Stiff-person syndrome, Pernicious anaemia, Vogt-Koyanagi-Harada syndrome, Primary biliary cirrhosis, Autoimmune hepatitis type I, Autoimmune hepatitis type II, Systemic lupus erythematosus, Rheumatoid arthritis, Polymyositis/ Dermatomyositis, Sjögren syndrome, Scleroderma, Vitiligo and Alopecia areata.

More preferably, the autoimmune disease is selected from the group comprising Pemphigus vulgaris, Goodpasture syndrome, Microscopic polyangiitis, Thrombotic thrombocytopenic purpura, Multiple sclerosis, Neuromyelitis optica, Type I diabetes, Narcolepsy with or without cataplexy, Celiac disease, Autoimmune Addison's Disease, Autoimmune haemolytic anaemia and Acquired hemophilia A.

According to the present invention, the term "treating" is used to refer to the alleviation of symptoms of a particular disease in a subject, and/or improvement of an ascertainable measurement associated with a particular disorder.

### The method of producing the amphiphilic polymer and the nanoparticles

Methods of producing the amphiphilic polymer and nanoparticles comprising the same are illustrated in Examples 1-4.

One method of obtaining the amphiphilic polymer with a number average molecular weight (Mn) of 6,000 to 1,000 g/mol resides in synthesizing the same using a two step method, comprising a step of producing a polymer of the anhydride and a step of hydrolyzing the anhydride to obtain an acid. The step of hydrolyzing the anhydride form of the polymer to obtain an acidic form can be illustrated as follows:

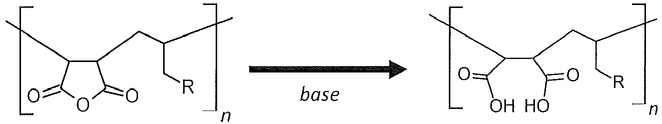

The present invention also provides a method of producing a nanoparticle comprising:
i) obtaining an amphiphilic polymer with a number average molecular weight (Mn) of 6,000 to 1,000 g/mol,
ii) optionally purifying the amphiphilic polymer,
iii) forming micelles of the amphiphilic polymer, and
iv) adding at least one peptide to form the nanoparticles.

In embodiments of the present invention in which the peptides are encapsulated by the micelle, step iv) is performed prior to step iii). In these embodiments, the peptides are added to the amphiphilic polymer prior to micelle formation.

The amphiphilic polymer used in the nanoparticles of the present invention may be prepared (step i) by a radical copolymerization using a radical initiator.

The molecular weight of the polymer can be controlled by varying the concentrations of the reactants or the amount of radical initiator. The molecular weight of the polymer can be analyzed by gel permeation chromatography.

The copolymerization may be conducted in an organic solvent such as 1,4 dioxane, xylene or chlorobenzene.

Many radical initiators are known in the art; they include various peroxides and azo-type compounds. Examples of suitable peroxides are benzoyl peroxide, lauryl peroxide, di-t-butyl peroxide, 2,4-dichlorobenzyl peroxide, t-butyl hydroperoxide, cumene hydroperoxide, diacetyl peroxide, diethyl peroxycarbonate, t-butyl perbenzoate and perborates. Suitable azo-type compounds include 2,2'-Azobis(2-methylpropionitrile), p-bromobenzenediazonium fluoborate, p-tolyldiazoaminobenzene, p-bromobenzenediazonium hydroxide, azomethane and phenyl-diazonium halides. Preferably, the radical initiator is 2,2'-Azobis(2-methylpropionitrile).

The copolymerization may be conducted at elevated temperatures such as from 70 to 120°C, preferably from 90 to 110°C.

Preferably, the copolymerization is initiated by heating the mixture to 70 to 120°C, preferably from 90 to 110°C.

In a preferred embodiment of the method of the present invention, step i) comprises the steps of mixing the reactants, deoxygenizing the mixture, heating the mixture and then cooling the mixture. Afterwards, the polymer may be dissolved and stirred overnight. The formed solid may be recovered, preferably using centrifugation.

In a preferred embodiment of the method of the present invention, step ii) includes the addition of a base to the polymer (*e.g*. NaOH). Preferably, the base is reacted with the polymer at elevated temperature, preferably between 50°C and 70°C, such as 60°C until almost all solids is dissolved. The resulting suspension may be acidified (*e.g.* pH <2). Afterwards, the reaction mixture may be extracted with an organic solvent such as ethyl acetate. The organic layer may be extracted with a sodium hydroxide solution. The aqueous solution may be again extracted with an organic solvent such as ethyl acetate and then dried to obtain the purified amphiphilic polymer.

The polymer may be further purified (step iii). Preferably, the polymer is further purified by extracting the polymer with n-hexane or n-heptane. The extraction can be performed at concentrations of greater than 10 g/L, preferably 100 g/l.

Furthermore, an additional purification step of the amphiphilic polymer may be added between steps i) and ii). In this additional purification step, the crude reaction product of the polymerization is dissolved and precipitated. In a preferred embodiment, the solvent is dichloromethane and the polymer is precipitated using a mixture of methanol/heptane or acetonitrile/iso-propanol. The mixtures used may contain for example 95/5% (v/v%) methanol/heptane, 10/90 (v/v%) acetonitrile/iso-propanol or 5/95 (v/v%) acetonitrile/isopropanol. In a preferred embodiment, the precipitation mixture is added at temperatures of -10 to 10°C, preferably -5 to 5°C.

The purity of the amphiphilic polymer after hydrolysis and workup can be measured by ¹H NMR.

In the method of the present invention, the micelle is formed by forming a solution containing the amphiphilic polymer. Preferably, the micelle is formed in an aqueous solution. Co-stabilizers may be added to the amphiphilic polymer to improve micelle formation.

The peptides to be used in step iv) may be synthesized using state of the art solid phase chemistry.

The peptides may be covalently linked to the micelle or non-covalently associated.

In a preferred embodiment of the method of the present invention, the peptides are coupled to the surface of the nanoparticle using peptide coupling techniques known in the art, *e.g.,* carbodiimide or succinimide coupling.

In a specifically preferred embodiment of the method of the present invention, the peptides are coupled to the surface of the nanoparticle via EDC chemistry (1-Ethyl-3-(3-dimethylaminopropyl)carbodiimide) in aqueous phase.

The resulting nanoparticles may be purified using intensive washing and filtration steps to remove the coupling reagent(s) and any low molecular weight components.

In a preferred embodiment, the method of producing a nanoparticle comprises:
a) obtaining a hydrophobic core nanoparticle,
b) obtaining an amphiphilic polymer with a number average molecular weight (Mn) of 6,000 to 1,000 g/mol, preferably using radical copolymerization,
c) optionally purifying the amphiphilic polymer,
d) mixing of the hydrophobic core nanoparticles and the amphiphilic polymer to form micelles,
e) adding at least one peptide to form the nanoparticles.

The discussion of steps i) to iii) and iv) above also applies to steps b) to d) and e) respectively.

The hydrophobic core of step a) can be synthesized using appropriate reactants in solution. In a preferred embodiment of the method of the present invention, the hydrophobic core can be synthesized using metal salts and salts of carboxylic acids as reactants in the presence of organic solvents. Preferably, the reaction is conducted at elevated temperatures under oxygen restriction.

The micelle can be formed by the arrangement of the amphiphilic copolymer around the core in step e). Preferably, step d) comprises the sub-steps of solubilising the amphiphilic polymer and the core particles, removing the solvent until a thin film is formed, adding a basic aqueous solution at increased temperature and ambient pressure to form an aqueous colloidal dispersion, diluting the solution and optionally filtering it. Afterwards, several washing steps may be applied.

### Examples

The invention is illustrated by the following examples, which describe in detail the synthesis of nanoparticles according to the present invention.

These examples should not be considered as limiting the scope of the invention, but as illustrating it.

### Example 1: Preparation of superparamagnetic iron oxide crystalline cores (SPIONs)

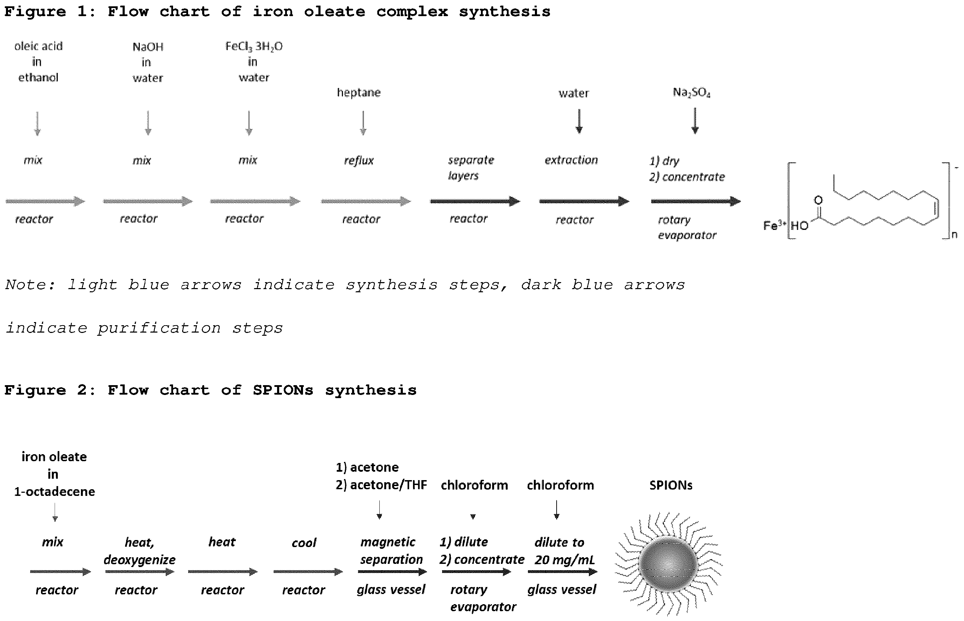

The synthesis of an iron oleate complex is schematically shown in Figure 1.

In a first step, an iron oleate complex was synthesized by mixing oleic acid, sodium hydroxide and iron chloride under reflux at 70°C. The product was purified by several washing steps in a separation funnel, and then dried with sodium sulphate and concentrated in a rotary evaporator. This resulted in iron oxide crystalline cores (Figure 1).

In a second step (Figure 2), the iron oleate complex was dissolved in 1-octadecene at room temperature and stirred until complete dissolution. Then, oleic acid was added, deoxygenated and heated for 3 hours at 300°C for the formation of iron oxide nanocrystals.

After cooling, the product was purified by several washing steps using magnetic separation and acetone/tetrahydrofuran (THF). Purified SPIONS were diluted in chloroform and concentrated in a rotary evaporator resulting in SPIONs with narrow size distribution and good crystallinity.

### Example 2: Preparation of low molecular weight poly(maleic acid-alt-1-octadecene) (LM-PMAcOD)

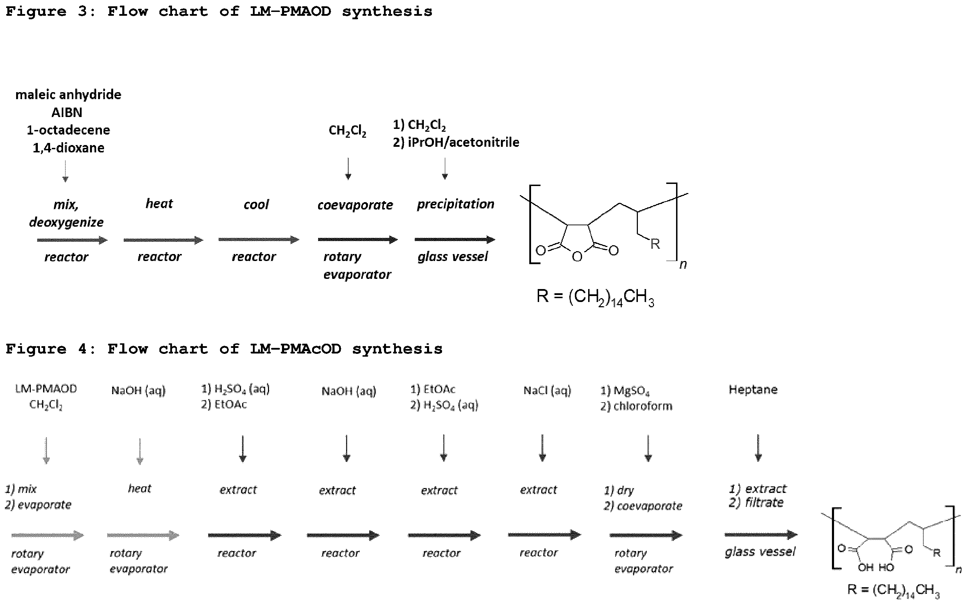

The synthesis of low molecular weight poly(maleic acid-alt-1-octadecene) (LM-PMAcOD) was achieved in a two-step process which is schematically shown in Figures 3 and 4.

In a first step, maleic anhydride (48.9 mmol) and 1-octadecene (48.2 mmol) were dissolved in 10 ml 1,4 dioxane (inhibitor of 1,4-dioxane was previously removed by filtering it over 3g aluminum oxide). Afterwards, 5.79 mmol AIBN (2,2'-Azobis(2-methylpropionitrile)) was added. The flask was equipped with a cooler and subsequently flushed with nitrogen and kept on a nitrogen overpressure. The mixture was heated to 100°C while stirring. 1 hour after the heating was started, the heating plate was removed together with the stoppers; exposing the reaction to air. The mixture was cooled to room temperature for two days while stirring. The product was purified by coevaporation with dichloromethane and precipitated with isopropanol and acetonitrile. Low molecular weight poly(maleic anhydride-*alt*-1-octadecene) (LM-PMAOD) with a number average molecular weight (Mn) of 2,500 to 4,000 g/mol was obtained.

In a second step, LM-PMAOD was hydrolysed to poly(maleic acid-*alt*-1-octadecene) (LM-PMAcOD) in sodium hydroxide solution. An acid-base extraction with H₂SO₄, ethylacetate, and NaOH was performed for the purification of the product and to remove impurities such as residual 1-octadecene. The product was dried over magnesium sulphate, co-evaporated with chloroform and finally purified by solid-liquid extraction in n-heptane (Figure 4).

The number average molar mass (Mn) and the mass average molar mass (Mw) of the polymer obtained was determined using gel permeation chromatography.

A sample of 1.5 mg of LM-PMAcOD was dissolved in 1.0 mL of THF (devoid of stabilizer) and submitted to GPC. Polystyrene was used as calibration standard. Tetrahydrofuran was used as a eluent and the flow rate was 1 ml/min. The temperature was set to 30°C.

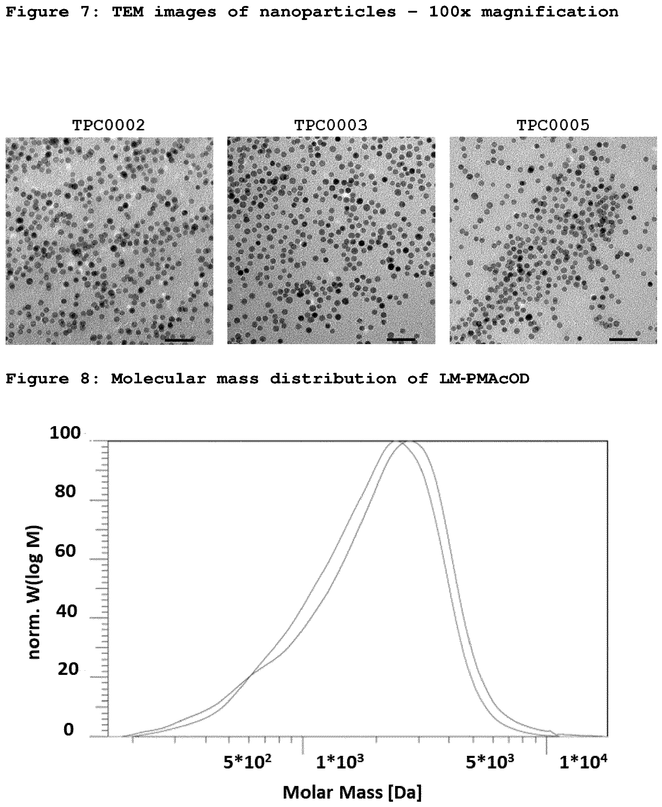

The number average molar mass (Mn) of the LM-PMAcOD produced was 1540 g/mol and the mass average molar mass (Mw) was 2410 g/mol. Hence, a PDI value of 1.56 was calculated for the sample, which is characteristic for a polymer produced via non-controlled free radical polymerization (see Figure 8).

### Example 3: Polymer coating of SPIONs

The polymer coating of SPIONs is schematically shown in Figure 5.

Example 3a: 100 mg LM-PMAcOD obtained in Example 2 was dissolved in 4 mL chloroform in a 100 mL round bottomed flask. The mixture was heated until the polymer was fully dissolved. 3.3 mL of the oleate-SPION solution obtained in Example 1 was added to the mixture and subsequently evaporated at <10 mbar for 15 minutes at 40°C on the rotavap with 280 RPM. Then, 10 mL 5 mM NaOH was added to the mixture and stirred on the rotavap for 15 minutes at 50°C until all black solids were dissolved. The solution was diluted 8 times using 70 mL 25 mM NaOH to dissolve the entire polymer. The obtained solution was stirred on the rotavap for 15 minutes, resulting in a brown solution.

The product was filtered over a 0.45 um and a 0.2 µm. PES filter. Afterwards, the probe was purified by tangential flow filtration (TFF).

Example 3b: The same procedure was performed with commercially available 30-50 kDa polymer (#419117 Merck) after hydrolization according to Example 2. As the removal of the unbound polymer from the polymer coated SPIONs by TFF was insufficient, additional magnetic separation was carried out using a Miltenyi column to purify the PMAcOD-SPION batch MX0194.

### Example 4: Peptides and peptide coupling

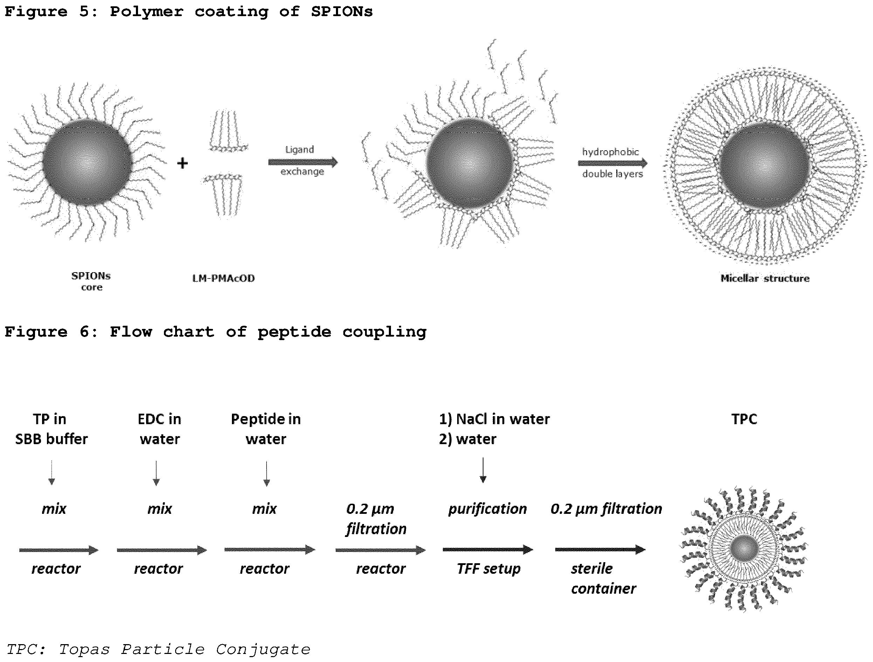

The peptide coupling and nanoparticle synthesis is schematically shown in _Figure 6.

The synthesis of the peptides was accomplished via Fmoc chemistry from the C to N direction using solid phase peptide synthesis (SPPS). The alpha amino group of each amino acid was protected with a fluoren-9-ylmethoxycarbonyl (Fmoc) group, while side chain functional groups were also blocked with various appropriate protective groups.

In general, the SPPS consists of repeated cycles of N-terminal deprotection followed by coupling reactions. The first Fmoc-protected amino acid was coupled to the resin. Afterwards, the amine group was deprotected with a mixture of piperidine in dimethylformamide (DMF), and then coupled with the free acid of the second Fmoc-protected amino acid. The cycle was repeated until the desired sequence was obtained. The resin was washed between each step. The completion of each coupling reaction was monitored by a qualitative ninhydrin test. In the last step of the synthesis, the crude peptide-resin was successively washed with DMF and methanol, and dried. Then, the protective groups were removed from the peptide and the peptide was cleaved from the resin using trifluoroacetic acid (TFA). The obtained crude peptide was isolated by ether precipitation from the cleavage mixture.

Further, the peptide was purified through preparative HPLC to reach purity requirements, and the counter ion TFA was replaced with chloride by using an appropriate solvent-buffer system. Finally, the purified peptide was lyophilized.

The peptides used have an amino acid at the N-terminus and a free acid (HCl salt) at the C-terminus and have a length of 15 amino acids.

Characterization of the free peptides (starting materials) was performed by LC-MS.

The peptide sequences and calculated monoisotopic mass used in the examples of the present application are shown in Table 1.

**Table 1: Peptide sequences and monoisotopic mass**

| **SEQ ID NO:** | **Sequence** | **Molecular formula** | **Monoisotopic mass (theoretical) (Da)** |
|---|---|---|---|
| 1 | LNSKIAFKIVSQEPA | C₇₅H₁₂₅N₁₉O₂₂ | 1643.9 |
| 2 | TPMFLLSRNTGEVRT | C₇₄H₁₂₄N₂₂O₂₃S | 1720.9 |
| 3 | REGIAFRPASKTFTV | C₇₆H₁₂₂N₂₂O₂₁ | 1678.9 |

The molecular weight of the peptides was measured by multimode electrospray atmospheric pressure chemical ionization mass spectrometry.

The peptides were coupled to the surface of the micelle obtained in Example 3a using 1-ethyl-3-(3-dimethylaminopropyl)carbodiimide (EDC) chemistry in boric acid/sodium tetraborate decahydrate (SBB) buffer.

EDC in SBB buffer was added to the micelle obtained in Example 3. After 15 Minutes at RT, the peptides were added and the reaction mixture was stirred for 2 hours and 15 minutes at RT.

The resulting nanoparticle solution was filtered and purified by tangential flow filtration (TFF) purification.

### Example 5: Characterization of the nanoparticles

The nanoparticles obtained in Example 4 were further characterized using a variety of analytical methods.

Characterization of the iron oxide core was performed using TEM and SAXS. TEM measurements were performed on the nanoparticles dispersed in 5% (w/v) D-mannitol, 5 mM TRIS and 6 mM L-lactic acid.

The calculated particle size results based on TEM analysis of the nanoparticles produced in Example 4 are summarized in Table 2. Representative TEM images are shown in Figure 7.

**Table 2: Average iron oxide core size results based on TEM analysis**

| **TPC** | **Average iron oxide core size (nm)** | **Standard deviation (%)** |
|---|---|---|
| TPC0002 | 9.74 | 1.04 |
| SEQ ID NO:1 | | |
| TPC0003 | 9.45 | 0.92 |
| SEQ ID NO:2 | | |
| TPC0005 | 9.80 | 0.96 |
| SEQ ID NO:3 | | |

SAXS measurements were performed on the nanoparticles dispersed in 5% (w/v) D-mannitol, 5 mM TRIS and 6 mM L-lactic acid.

The calculated particle size results based on SAXS analysis of the nanoparticles produced in Example 4 are summarized in Table 3.

**Table 3: Average iron oxide core size results based on SAXS analysis**

| TPC | Average iron oxide core size (nm) | Standard deviation (%) |
|---|---|---|
| SEQ ID NO: | | |
| TPC0002 | 9.8 | 1.1 |
| SEQ ID NO:1 | | |
| TPC0003 | 10.0 | 1.1 |
| SEQ ID NO:2 | | |
| TPC0005 | 9.8 | 1.1 |
| SEQ ID NO:3 | | |

Characterization of particle size and distribution was performed by dynamic light scattering (DLS). The hydrodynamic diameter (z-average) and polydispersity index were determined using a Malvern Zetasizer Nano ZS or equivalent in unimodal mode.

These measurements were performed on the nanoparticles produced in Example 4 dispersed in 5% (w/v) D-mannitol, 5 mM TRIS and 6 mM L-lactic acid.

The results are summarized in Table 4. From these results, it can be observed that the formulation stabilizes the particles and can reduce the percentage of large particles.

**Table 4: Size distribution results based on DLS analysis**

| TPC SEQ ID NO: | z-average (nm) | D₁₀ (nm) | D₅₀ (nm) | D₉₀ (nm) | polydispersity index (%) |
|---|---|---|---|---|---|
| TPC0002 SEQ ID NO:1 | 23 | 15.8 | 24.0 | 37.6 | 0.13 |
| TPC0003 SEQ ID NO:2 | 25 | 16.8 | 25.1 | 39.3 | 0.17 |
| TPC0005 SEQ ID NO:3 | 26 | 16.4 | 24.9 | 53.9 | 0.25 |

The surface charge of the particles was analyzed by measuring the zeta potential at pH 6 to 7 (pH during measurement) using a Malvern Zetasizer Nano ZS instrument. These measurements were performed on the nanoparticles produced in Example 4 dispersed in 5% (w/v) D-mannitol, 5 mM TRIS and 6 mM L-lactic acid.

The results are summarized in Table 4.

**Table 4: Zeta potential results**

| TPC SEQ ID NO: | Zeta potential (mV) | Zeta deviation (mV) | Conductivity (mS/cm) | Result quality |
|---|---|---|---|---|
| TPC0002 SEQ ID NO:1 | -39.5 | 5.38 | 1.30 | Good |
| TPC0003 SEQ ID NO:2 | -39.1 | 5.95 | 1.44 | Good |
| TPC0005 SEQ ID NO:3 | -30.1 | 4.63 | 1.42 | Good |

The total polymer content was determined using GPC. The peptides were hydrolyzed, and the particles destroyed in 6 M HCl. The PMAcOD was extracted with ethyl acetate after addition of EDTA. After evaporation of solvent, the residue was re-dissolved in a THF/acetic acid mixture before analysis.

The results are summarized in Table 5.

**Table 5: Total polymer content**

| TPC SEQ ID NO: | Total polymer content (mg/mL) |
|---|---|
| TPC0002 SEQ ID NO:1 | 1.62 |
| TPC0003 SEQ ID NO:2 | 1.42 |
| TPC0005 SEQ ID NO:3 | 1.36 |

The spectroscopic properties of the nanoparticles were determined by Fourier-transform infrared spectroscopy (FTIR).

The assignments of the major absorption bands are summarized in Table 6.

**Table 6: Proposed description of IR vibrational modes for the nanoparticles**

| **Frequency of absorption bands (cm⁻¹)** | **Description of the IR vibrational modes** |
|---|---|
| 3500 - 3000 | Broad, carboxylic acid O-H stretch |
| 2920-2919, 2851-2850 | Alkane C-H stretch |
| 1646-1642 | C=O stretch, weakly coupled to C-N stretch and N-H stretch |
| 1534-1544 | C-N stretch, strongly coupled with N-H bending |
| 1402-1395 | Carboxylic acid O-H bending |
| 574-569 | Fe-O bond |

### Example 6: Effect of different sizes of amphiphilic polymer

The effect of different sizes of amphiphilic polymer in the nanoparticles of the present invention was studied. SPIONs were coated with PMAcOD polymers with a number average molecular weight of 3100, 4800 and 5900 g/mol.

These polymers were synthesized, purified and characterized, after which they were employed in a coating reaction of oleate-SPIONs. After coating the excess polymer was removed using TFF purification.

### a) Synthesis of poly(maleic acid-alt-1-octadecene) (PMAcOD) with different molecular weights

Three different lengths of poly(maleic acid-*alt*-1-octadecene) were synthesized by free radical polymerization of maleic anhydride and 1-octadecene. The reaction was carried out in 1,4-dioxane using 2,2'-Azobis(2-methylpropionitrile) as initiator. Polymerization was initiated by heating the mixture to 100°C. Secondly, after purification, the polymer was hydrolyzed using a sodium hydroxide solution to obtain PMAcOD.

The polymers with higher molecular weight were synthesized by performing the polymerization under more concentrated conditions and/or executing the polymerization with a decreased amount of initiator.

The amount of reactants used in the synthesis of the three polymers is summarized in Table 7.

**Table 7: Amount of reactants**

| | Length (g/mol) | Maleic anhydride (g) | 1-octadecene (g) | AIBN (g) | 1,4-dioxane (mL) |
|---|---|---|---|---|---|
| PMAcOD3100 | 3100 | 4.8 | 12.36 | 1.004 | 40.8 |
| PMAcOD4800 | 4800 | 4.8 | 12.18 | 0.950 | 10.0 |
| PMAcOD5900 | 5900 | 4.8 | 12.18 | 0.500 | 10.0 |

The purity of the polymer after hydrolysis of the maleic anhydrides and workup was measured by ¹H-NMR (400 Hz, 30 mg sample, 10 mg benzoic acid standard, 700 µL D-chloroform) and the length of polymer was analyzed by gel permeation chromatography (Agilent PL-gel mixed-D, 300 x 7.5 mm ID, 5 µm, 2 mg/mL in THF/acetic acid (90/10), 15 min run).

### b) Effect of different lengths of amphiphilic polymer

The PMAcOD with various lengths were used to coat oleate-SPIONs.

First, the coatings were performed using the same polymer-to-SPION weight ratio. To find the best coating conditions, the polymer-to-SPION molar ratio was kept constant. The amounts of aggregates were compared. The method with the least aggregates was repeated and subsequently purified using TFF. The removal of polymer and the amount of aggregates was monitored during TFF using size exclusion chromatography (SEC).

Oleate-SPIONs were coated with PMAcOD3100, PMAcOD4800 and PMAcOD5900) to determine the optimal polymer/SPION ratio.

To coat the oleate-SPIONs the polymer and the nanoparticles were dissolved in chloroform. Subsequently, the chloroform was evaporated. In the final step of the coating procedure, an aqueous sodium hydroxide solution was added to the flask and mixed at 50°C. For PMAcOD3100 100 mg polymer was used. For PMAcOD4800 coatings with 100 mg polymer and 145 mg polymer were compared. For PMAcOD5900 coatings with 100 mg polymer and 177 mg polymer were compared. This way, equal amounts of monomer were compared to equal amounts of polymer chains with respect to PMAcOD3100.

By using size exclusion chromatography, it was determined which condition resulted in the least amount of aggregates.

The results are summarized in Table 8.

**Table 8: Overview of aggregates produced by the coating of SPIONs with different polymers**

| | Length (g/mol) | Amount of polymer (mg) | Equivalents of polymer compared to PMAcOD3100 | Aggregates (%) |
|---|---|---|---|---|
| PMAcOD3100 | 3100 | 100 | 1 | 5.9 |
| PMAcOD4800 | 4800 | 100 | 0.65 | 5.2 |
| | | 145 | 0.93 | 5.9 |
| PMAcOD5900 | 5900 | 100 | 0.53 | 16.0 |
| | | 177 | 0.93 | 7.8 |

### c) Effect of different polymer lengths after purification

Oleate-SPIONs were coated using the same protocol as used before. For all polymers the same amount of polymer chains was used. After coating, the PMAcOD-SPIONs were purified by TFF. Due to the difficulty of removing the final amounts of polymer PMAcOD5900, it was decided to also try this coating with 0.15 g polymer (0.79 equivalents compared to standard experiment with PMAcOD3100).

Using SEC it was determined how many aggregates and how much polymer was left in the sample. After coating, PMAcOD3100 comprised 5.9% aggregates, PMAcOD4800 comprised 6.2% aggregates and PMAcOD5900 comprised 10.7% aggregates. This matched with the data described in the previous section.

Next, the PMAcOD-SPIONs were purified by TFF. The particles were loaded onto the membrane, after which they were rinsed with 5 mM NaOH/45 mM NaCl. Every 10 DVs (for PMAcOD3100) or 20 DVs (for PMAcO4800 and PMAcOD5900) (DV = diafiltration volume in the TFF purification), a sample was taken and measured by SEC (afterwards). During the TFF purification, also in-process-samples were taken to keep track of the decrease in polymer and increase in aggregates.

The results are summarized in Table 9.

**Table 9: Overview of TFF experiments**

| Run | | Amount of polymer (mg) | Aggregates at start (%) | Aggregates after TFF (%) | DVs used |
|---|---|---|---|---|---|
| 1 | PMAcOD3100 | 100 | 5.9 | 7.0 | 160 |
| 2 | PMAcOD4800 | 100 | 6.2 | 7.3 | 160 |
| 3 | PMAcOD5900 | 177 | 10.7 | 10.7 | 150 |
| 4 | | 150 | 10.9 | 10.4 | 110 |

The TFF products of runs 1, 2 and 4 were filtered over a 0.2 µm filter. Run 1 was also filtered over a 0.1 um filter to decrease the amount of aggregates.

Before and after filtration of the samples, dynamic light scattering (DLS) measurements were performed to determine the Z-average diameter and the polydispersity index (PDI). All batches of PMAcOD-coated SPIONs showed a Z-average diameter of 26 to 28 nm with a PDI of 0.25 to 0.33 (see Table 11).

The amounts of iron were determined using atomic absorption spectroscopy (AAS) to be 38 to 50 mg after filtration, which corresponds to an iron recovery of 83 to 110 %.

The results are summarized in Table 10.

**Table 10: DLS data; before and after filtration.**

| Run | Product | Unfiltered | | Filtered | | | |
|---|---|---|---|---|---|---|---|
| | | Z-average (nm) | PDI | Z-average (nm) | PDI | Iron content (mg) | Iron recovery (%) |
| 1 | PMAcOD3100 - SPIONS | 31.7 | 0.382 | 26.8 | 0.256 | 38 | 83 |
| 2 | PMAcOD4800 - SPIONS | 28.8 | 0.360 | 27.0 | 0.325 | 50 | 110 |
| 3 | PMAcOD5900 - SPIONS | 32.1 | 0.367 | 27.6 | 0.251 | 46 | 101 |

### Example 7: Comparison of purification of PMAcOD-SPIONs with polymers having different molecular weight

SPIONs coated with a commercial high molecular weight PMAcOD (Mn of 30,000 to 50,000 g/mol) and a low molecular weight PMAcOD (Mn of 3,000 to 5,000 g/mol) were produced as described in Examples 1-4. Afterwards, tangential flow filtration (TFF) purification was used to remove unbound material. TFF is the method of choice for the purification of the coated SPIONS because it can be scaled easily. Size exclusion chromatography (SEC) analysis of retentate and permeate samples was performed to monitor the TFF-purification process. The purification efficiency using TFF of the SPIONs coated with high and low molecular weight polymers was compared.

### a) Purification of high molecular weight PMAcOD-SPIONs

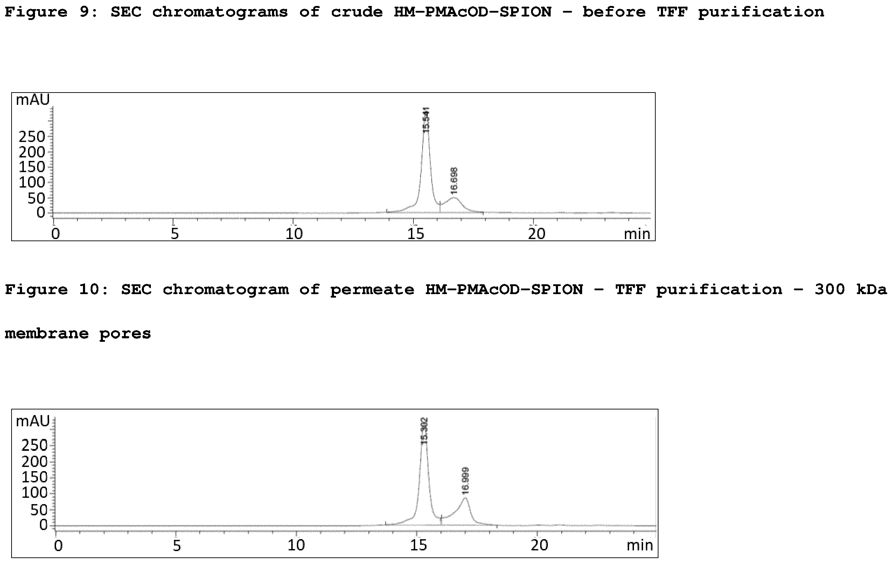

In a first step, a sample of the crude HM-PMAcOD-SPIONs was analyzed using SEC prior to purification by TFF (crude sample, see Figure 9). The batch of crude HM-PMAcOD-SPIONs showed a small shoulder left of the main HM-PMAcOD-SPION peak (RT: 15.541), indicating that this batch contained a minimal amount of large aggregates, with 21.2% (a/a) of unbound polymer free (RT: 16.698) in the dispersion.

Subsequently, the high molecular weight PMAcOD-SPION sample was filtered by TFF using a 300 kDa TFF membrane in order to separate the HM-PMAcOD-SPIONs after polymer coating from unbound polymer molecules. As illustrated in Figures 9 and 10 no separation was achieved but both HM-PMAcOD-SPIONs and HM-PMAcOD polymer was contained in the permeate after filtration.

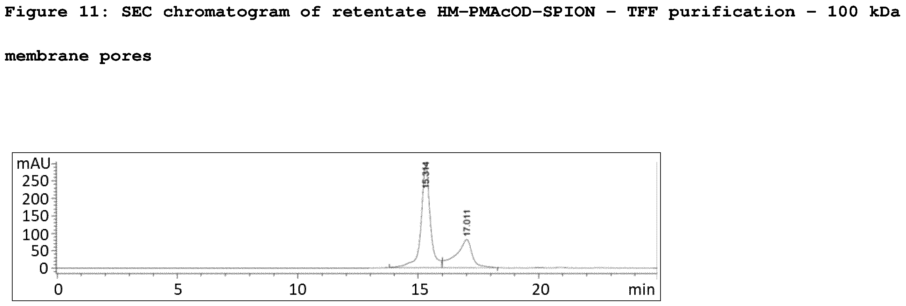

Thus, several options were tested to see if this could be reduced. However, none succeeded. The retention of the HM-PMAcOD-SPIONs was too low to obtain a good separation between SPIONs and polymer without losing too much product. A membrane with a decreased pore size of 100 kDa allowed retention of the polymer coated SPIONs but at the same time led to retention and concentration of the polymer (illustrated in Figure 11).

In conclusion, it has been found that neither filtration with a 300 kDa TFF membrane nor filtration with a 100 kDa TFF membrane allows for the purification of HM-PMAcOD-SPIONs from unbound HM-PMAcOD material.

### b) Purification of low molecular weight PMAcOD-coated SPIONs

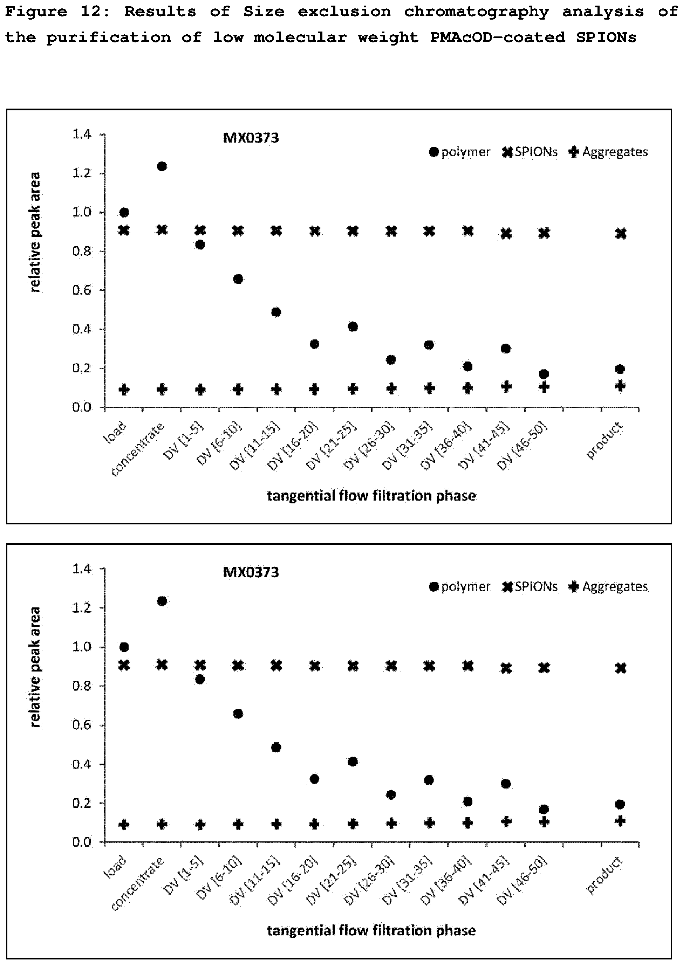

In a second approach, the low molecular weight PMAcOD-coated SPIONs were purified using TFF (100 kDa filter membrane). Again, size exclusion chromatography analysis of retentate and permeate samples was performed to monitor the TFF purification process. Results are shown in Figure 12 for two batches (batches MX0373A and MX0374A).

The use of LM-PMAcOD (Mn of 3,000 to 5,000 g/mol) showed efficient diffusion of the unbound polymer over a 100 kDa TFF membrane and removal of more than 90% of unbound polymer from the LM-PMAcOD-SPIONs.

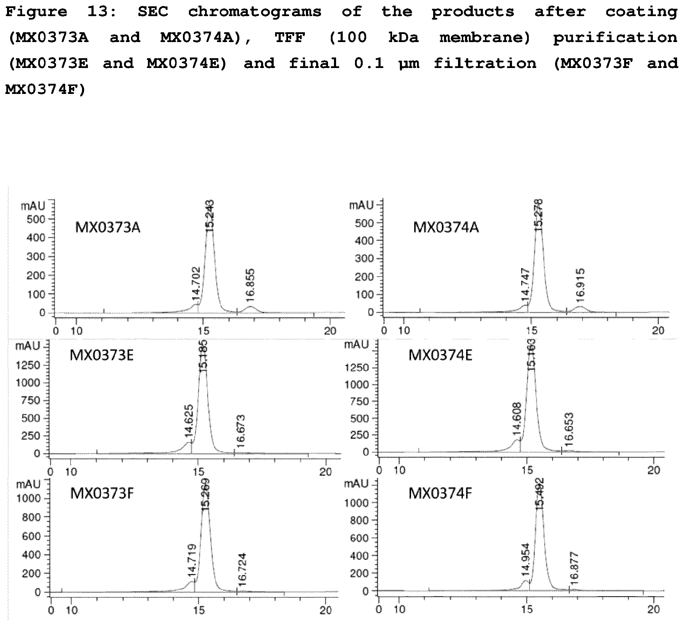

The SEC chromatograms of the products after coating (MX0373A and MX0374A), TFF (100 kDa membrane) purification (MX0373E and MX0374E) and final 0.1 um filtration (MX0373F and MX0374F) are shown in Figure 13.

Furthermore, the SEC data of samples after TFF purification (100 kDa filter membrane; batches MX0373E and MX0374E) and after TFF purification followed by 0.1 µm filtration (batches MX0373F and MX0374F) is shown in Table 12 below.

**Table 12:**

| Product | % SPIONs (a/a) | % Aggregates (a/a) | % un-bound polymer removed |
|---|---|---|---|
| MX0373E | 89.0 | 11.0 | 80.4 |
| MX0373F | 89.5 | 10.5 | |
| MX0374E | 88.7 | 11.3 | 78.9 |
| MX0374F | 89.2 | 10.8 | |

As can be seen from the data provided, it was possible to efficiently purify the LM-PMAcOD-SPIONs by TFF, whereas the purification of the HM- PMAcOD-SPIONs was inefficient and did not result in a pure product.

### Example 8: In-vivo safety after intravenous injection in mice of low molecular weight polymer based micelles containing an iron oxide core compared to high molecular weight polymer based micelles containing an iron oxide core

**a)** The batch MX0194 produced in Example 3b was injected intravenously in female CD1 mice at a dose of 1 mmol Fe/kg bw and 2 mmol Fe/kg bw. One mouse died at 2 mmol Fe/kg which was defined to be test item related. Mice were euthanized 14 d after injection. Significant increases in absolute organ weight compared to matched controls were found (see table 13) which was defined to be an adverse effect of test item injection.
**b)** A PMAcOD-SPION batch produced according to Example 3a was injected intravenously in female CD1 mice 3 times at a dose of 1 mmol Fe/kg bw (3 mmol Fe/kg bw in total) with 14 days between injection 1 and 2 and injection 2 and 3. Animals were euthanized 24 hours after the last injection. Only a slight but not significant increase in liver weight was found and no increase in lung weight (see table 13).

The influence on organ weight by high molecular weight polymer based nanoparticles is related to a long-term adverse effect of the high molecular weight polymer due to the fact that the iron core was similar for the batches produced in Example 8a and 8b.

**The results of the comparative study are summarized in Table 13.**

**Table 13:**

| | liver weight increase [weight %] | lung weight increase [weight %] |
|---|---|---|
| example 8a 1 mmol Fe/kg | 17.4* | 2.9 |
| n=5 | | |
| example 8a 2 mmol Fe/kg | 25.0** | 26.2** |
| n=4 | | |
| example 8b 3 mmol Fe/kg | 5.7 | 0.7 |
| n=12 | | |
| organ weight increase is calculated from absolute organ weight were absolute organ weight of body weight matching control animals is given as 100%. | | |
| *p<0.05; **p<0.01 One Way Anova with Dunnett | | |

The low molecular weight polymer was thus shown to have a lower toxicity than the high molecular weight polymer.

## Claims

1. A nanoparticle comprising
a) a micelle comprising an amphiphilic polymer with a number average molecular weight (Mn) of 6,000 to 1,000 g/mol, and
b) at least one peptide comprising at least one T cell epitope.

2. The nanoparticle of claim 1, wherein the nanoparticle further comprises a solid hydrophobic core which is at least partically coated by the micelle, wherein the core comprises a traceable inorganic material selected from the group comprising iron oxide, CdSe/CdS/ZnS, silver and gold.

3. The nanoparticle of claims 1 or 2, wherein the peptide is associated with the outside of the micelle.

4. The nanoparticle of the preceding claims, wherein the amphiphilic polymer has a main hydrophilic poly-maleic anhydride backbone having hydrophobic alkyl side chains.

5. The nanoparticle of the preceding claims, wherein the amphiphilic polymer comprises the following building block 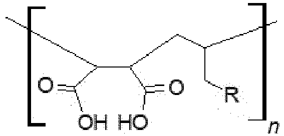 wherein R is a hydrocarbyl group or a substituted hydrocarbyl group, preferably R is a C₄ to C₂₂ alkyl group, preferably C₈ to C₂₀ alkyl group.

6. The nanoparticle of claim 5, wherein R is a linear alkyl group, preferably a linear C₁₁ to C₁₇ alkyl group, most preferably R is a linear pentadecyl group.

7. The nanoparticle of any of the preceding claims, wherein the amphiphilic polymer is selected from the group comprising poly(maleic acid-alt-1-octadecene), poly(maleic acid-alt-1-dodecene) and poly(maleic acid-alt-1-tetradecene), preferably the polymer is poly(maleic acid-alt-1-octadecene), and
the number average molecular weight of the polymer is from 6,000 to 1,000 g/mol.

8. The nanoparticle of any of the preceding claims, wherein the peptide is covalently linked to the micelle.

9. The nanoparticle of claim 8, wherein the peptide is covalently linked to the micelle using carbodiimide or succinimide coupling.

10. The nanoparticle of of any one of claims 1 to 7, wherein the peptide is non-covalently associated.

11. The nanoparticle of any of the preceding claims, wherein the peptide comprises 9 to 60, preferably from 10 to 20 amino acids.

12. The nanoparticle of any of the preceding claims, wherein the nanoparticle is negatively charged at a pH of 6 to 7.

13. The nanoparticle of any of the preceding claims, wherein the nanoparticle has a hydrodynamic diameter between 100 and 10 nm, preferably between 50 and 10 nm, more preferably between 20 and 40 m as measured by dynamic light scattering.

14. A pharmaceutical composition comprising the nanoparticle of claims 1 to 13.

15. A pharmaceutical composition for use in suppressing a specific immune response comprising the nanoparticle of claims 1 to 13.

16. The pharmaceutical composition for use in suppressing a specific immune response of claim 15, wherein said response is associated with an autoimmune disease, preferably with an autoimmune disease selected from the group comprising Pemphigus vulgaris, Pemphigus foliaceus, Epidermolysis bullosa Acquisita, Bullous pemphigoid, Cicatricial pemphigoid, Goodpasture syndrome, Microscopic polyangiitis, Granulomatosis with polyangiitis (Granulom. Wegener), Thrombotic thrombocytopenic purpura, Immune thrombocytopenic purpura, Uveitis, HLA-B27-associated acute anterior uveitis, Multiple sclerosis, Neuromyelitis optica, Type I diabetes, Narcolepsy with or without cataplexy, Celiac disease, Dermatitis herpetiformis, Allergic airways disease/Asthma, Myasthenia gravis, Hashimoto thyreoiditis, Autoimmune thyroid disease, Graves disease, Autoimmune thyroid disease, Autoimmune Hypoparathyroidism, Autoimmune thyroid disease, Antiphospholipid syndrome, Autoimmune Addison's Disease, Autoimmune haemolytic anaemia, Chronic inflammatory demyelinating, Polyneuropathy, Guillain-Barré syndrome, Autoimmune neutropenia, Linear morphea, Batten disease, Acquired hemophilia A, Relapsing polychondritis, Isaac's syndrome (acquired neuro-myotonia), Rasmussen encephalitis, Morvan syndrome, Stiff-person syndrome, Pernicious anaemia, Vogt-Koyanagi-Harada syndrome, Primary biliary cirrhosis, Autoimmune hepatitis type I, Autoimmune hepatitis type II, Systemic lupus erythematosus, Rheumatoid arthritis, Polymyositis/ Dermatomyositis, Sjögren syndrome, Scleroderma, Vitiligo and Alopecia areata.

17. A method of producing a nanoparticle comprising:
i) obtaining an amphiphilic polymer with a number average molecular weight (Mn) of 6,000 to 1,000 g/mol,
ii) optionally purifying the amphiphilic polymer,
iii) forming micelles of the amphiphilic polymer, and
iv) adding at least one peptide comprising at least one T cell epitope to form the nanoparticles.

18. The method of claim 17, wherein step i) is a radical copolymerization synthesis step, preferably using 2,2'-Azobis(2-methylpropionitrile) as a radical initiator.

## Patentansprüche

1. Nanopartikel, umfassend
a) eine Micelle, die ein amphiphiles Polymer mit einem Zahlenmittel des Molekulargewichts (Mn) von 6.000 bis 1.000 g/mol umfasst, und
b) mindestens ein Peptid, das mindestens ein T-Zell-Epitop enthält.

2. Nanopartikel gemäß Anspruch 1, wobei der Nanopartikel ferner einen festen hydrophoben Kern umfasst, der zumindest teilweise von der Micelle beschichtet ist, wobei der Kern ein rückführbares anorganisches Material umfasst, das aus der Gruppe ausgewählt ist, die Eisenoxid, CdSe/CdS/ZnS, Silber und Gold umfasst.

3. Nanopartikel gemäß Anspruch 1 oder 2, wobei das Peptid mit der Außenseite der Micelle assoziiert ist.

4. Nanopartikel gemäß einem der vorhergehenden Ansprüche, wobei das amphiphile Polymer ein hydrophiles Polymaleinsäureanhydrid-Hauptgerüst mit hydrophoben Alkylseitenketten aufweist.

5. Nanopartikel gemäß einem der vorhergehenden Ansprüche, wobei das amphiphile Polymer den folgenden Baustein umfasst 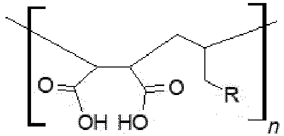 worin R eine Kohlenwasserstoffgruppe oder eine substituierte Kohlenwasserstoffgruppe ist, vorzugsweise ist R eine C₄ bis C₂₂ Alkylgruppe, vorzugsweise eine C₈ bis C₂₀ Alkylgruppe.

6. Nanopartikel gemäß Anspruch 5, wobei R eine lineare Alkylgruppe ist, vorzugsweise eine lineare C₁₁ bis C₁₇ Alkylgruppe, am meisten bevorzugt ist R eine lineare Pentadecylgruppe.

7. Nanopartikel gemäß einem der vorhergehenden Ansprüche, wobei das amphiphile Polymer aus der Gruppe ausgewählt ist, die Poly(maleinsäure-alt-1-octadecen), Poly(maleinsäure-alt-1-dodecen) und Poly(maleinsäure-alt-1-tetradecen) umfasst, vorzugsweise ist das Polymer Poly(maleinsäure-alt-1-octadecen), und
das Zahlenmittel des Molekulargewichts des Polymers liegt zwischen 6.000 und 1.000 g/mol.

8. Nanopartikel gemäß einem der vorhergehenden Ansprüche, wobei das Peptid kovalent an die Micelle gebunden ist.

9. Nanopartikel gemäß Anspruch 8, wobei das Peptid durch Carbodiimid- oder Succinimid-Kopplung kovalent an die Micelle gebunden ist.

10. Nanopartikel gemäß einem der Ansprüche 1 bis 7, wobei das Peptid nicht kovalent gebunden ist.

11. Nanopartikel gemäß einem der vorhergehenden Ansprüche, wobei das Peptid 9 bis 60, vorzugsweise 10 bis 20 Aminosäuren umfasst.

12. Nanopartikel gemäß einem der vorhergehenden Ansprüche, wobei das Nanopartikel bei einem pH-Wert von 6 bis 7 negativ geladen ist.

13. Nanopartikel gemäß einem der vorhergehenden Ansprüche, wobei das Nanopartikel einen hydrodynamischen Durchmesser zwischen 100 und 10 nm, vorzugsweise zwischen 50 und 10 nm, besonders bevorzugt zwischen 20 und 40 m hat, gemessen durch dynamische Lichtstreuung.

14. Pharmazeutische Zusammensetzung, die Nanopartikel gemäß einem der Ansprüche 1 bis 13 enthält.

15. Pharmazeutische Zusammensetzung zur Verwendung bei der Unterdrückung einer spezifischen Immunantwort, die Nanopartikel gemäß Anspruch 1 bis 13 umfasst.

16. Pharmazeutische Zusammensetzung zur Verwendung bei der Unterdrückung einer spezifischen Immunreaktion gemäß Anspruch 15, wobei die Reaktion mit einer Autoimmunerkrankung assoziiert ist, vorzugsweise mit einer Autoimmunerkrankung, ausgewählt aus der Gruppe, die Pemphigus vulgaris, Pemphigus foliaceus, Epidermolysis bullosa Acquisita, bullöses Pemphigoid, vernarbendes Pemphigoid, Goodpasture-Syndrom, mikroskopische Polyangiitis, Granulomatose mit Polyangiitis (Granulom. Wegener), thrombotische thrombozytopenische Purpura, immunthrombozytopenische Purpura, Uveitis, HLA-B27-assoziierte akute anteriore Uveitis, Multiple Sklerose, Neuromyelitis optica, Diabetes Typ I, Narkolepsie mit oder ohne Kataplexie, Zöliakie, Dermatitis herpetiformis, Allergische Atemwegserkrankung/Asthma, Myasthenia gravis, Hashimoto-Thyreoiditis, Autoimmunerkrankung der Schilddrüse, Morbus Basedow, Autoimmunerkrankung der Schilddrüse, Autoimmunhypoparathyreoidismus, Autoimmunerkrankung der Schilddrüse, Antiphospholipid-Syndrom, Autoimmunerkrankung von Addison, Autoimmunhämolytische Anämie, Chronisch entzündliche demyelinisierende Polyneuropathie, Guillain-Barré-Syndrom, Autoimmunneutropenie, lineare Morphea, Batten-Krankheit, erworbene Hämophilie A, rezidivierende Polychondritis, Isaac-Syndrom (erworbene Neuro-Myotonie), Rasmussen-Enzephalitis, Morvan-Syndrom, Stiff-Person-Syndrom, Perniziöse Anämie, Vogt-Koyanagi-Harada-Syndrom, Primär biliäre Zirrhose, Autoimmunhepatitis Typ I, Autoimmunhepatitis Typ II, Systemischer Lupus erythematodes, Rheumatoide Arthritis, Polymyositis/Dermatomyositis, Sjögren-Syndrom, Sklerodermie, Vitiligo und Alopecia areata umfasst.

17. Verfahren zur Herstellung eines Nanopartikels, wobei das Verfahren Schritte umfasst, bei denen man:
i) amphiphile Polymere mit einem Zahlenmittel des Molekulargewichts (Mn) von 6.000 bis 1.000 g/mol gewinnt,
ii) die amphiphilen Polymere gegebenenfalls reinigt,
iii) Micelle aus dem amphiphilen Polymer bildet, und
iv) mindestens ein Peptid zugibt, das mindestens ein T-Zell-Epitop umfasst, um die Nanopartikel zu bilden.

18. Verfahren gemäß Anspruch 17, bei dem Schritt i) ein radikalischer Copolymerisations-Syntheseschritt ist, vorzugsweise unter Verwendung von 2,2'-Azobis(2-methylpropionitril) als Radikalbildner.

## Revendications

1. Nanoparticule comprenant :
a) une micelle comprenant un polymère amphiphile dont la masse molaire moyenne en nombre (Mn) vaut de 6000 à 1000 g/mol,
b) et au moins un peptide comportant au moins un épitope pour lymphocyte T.

2. Nanoparticule conforme à la revendication 1, laquelle nanoparticule comprend en outre un noyau solide hydrophobe qui est, au moins en partie, enrobé par la micelle, lequel noyau comprend un matériau inorganique traçable choisi dans l'ensemble comprenant oxyde de fer, CdSe/CdS/ZnS, argent et or.

3. Nanoparticule conforme à la revendication 1 ou 2, dans laquelle le peptide est associé avec le côté externe de la micelle.

4. Nanoparticule conforme aux revendications précédentes, dans laquelle le polymère amphiphile comporte un squelette principal hydrophile de poly(anhydride maléique) qui porte des chaînes latérales alkyle hydrophobes.

5. Nanoparticule conforme aux revendications précédentes, dans laquelle le polymère amphiphile comporte le motif constitutif suivant : 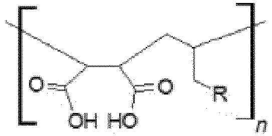 dans lequel R représente un groupe hydrocarbyle ou un groupe hydrocarbyle porteur de substituant, mais de préférence, R représente un groupe alkyle en C₄-C₂₂, et de préférence un groupe alkyle en C₈-C₂₀.

6. Nanoparticule conforme à la revendication 5, dans laquelle R représente un groupe alkyle linéaire, de préférence un groupe alkyle linéaire en C₁₁-C₁₇, le mieux étant que R représente un groupe penta-décyle linéaire.

7. Nanoparticule conforme à l'une des revendications précédentes, dans laquelle le polymère amphiphile est choisi dans l'ensemble comprenant les poly(acide maléique - alt - 1-octadécène), poly(acide maléique - alt - 1-dodécène) et poly(acide maléique - alt - 1-tétradé-cène), ce polymère étant de préférence un poly(acide maléique - alt - 1-octadécène), et la masse molaire moyenne en nombre de ce polymère vaut de 6000 à 1000 g/mol.

8. Nanoparticule conforme à l'une des revendications précédentes, dans laquelle le peptide est attaché à la micelle par liaison covalente.

9. Nanoparticule conforme à la revendication 8, dans laquelle le peptide est attaché à la micelle par liaison covalente, au moyen d'un couplage par carbodiimide ou par succinimide.

10. Nanoparticule conforme à l'une des revendications 1 à 7, dans laquelle le peptide est associé de manière non-covalente.

11. Nanoparticule conforme à l'une des revendications précédentes, dans laquelle le peptide comporte de 9 à 60 et de préférence de 10 à 20 résidus d'acides aminés.

12. Nanoparticule conforme à l'une des revendications précédentes, laquelle nanoparticule, à un pH de 6 à 7, porte une charge négative.

13. Nanoparticule conforme à l'une des revendications précédentes, laquelle nanoparticule présente un diamètre hydrodynamique qui, mesuré par diffusion dynamique de la lumière, vaut entre 100 et 10 nm, de préférence entre 50 et 10 nm, et mieux encore entre 20 et 40 nm.

14. Composition pharmaceutique comprenant des nanoparticules conformes aux revendications 1 à 13,

15. Composition pharmaceutique pour utilisation dans la suppression d'une réponse immunitaire spécifique, comprenant des nanoparticules conformes aux revendications 1 à 13.

16. Composition pharmaceutique pour utilisation dans la suppression d'une réponse immunitaire spécifique, conforme à la revendication 15, pour laquelle ladite réponse est associée à une maladie auto-immune, et de préférence, à une maladie auto-immune choisie dans l'ensemble comprenant les suivantes : pemphigus vulgaire, pemphigus foliacé, épidermolyse bulleuse acquise, pemphigoïde bulleuse, pemphigoïde cicatricielle, syndrome de Goodpasture, polyangéite microscopique, granulomatose avec polyangéite (granulomatose de Wegener), purpura thrombocytopénique thrombotique, purpura thrombocytopénique immun, uvéite, uvéite antérieure aiguë associée à HLA-B27, sclérose en plaques, neuromyélite optique aiguë, diabète de type I, narcolepsie avec ou sans cataplexie, maladie coeliaque, dermatite herpétiforme, asthme/maladie allergique des voies aériennes, myasthénie grave, thyroïdite chronique de Hashimoto, maladie thyroïdienne auto-immune, maladie de Basedow-Graves, maladie thyroïdienne auto-immune, hypoparathyroïdie auto-immune, maladie thyroïdienne auto-immune, syndrome des antiphospholipides, maladie auto-immune d'Addison, anémie hémolytique auto-immune, maladie démyélinisante inflammatoire chronique, polyneuropathie, syndrome de Guillain-Barré, neutropénie autoimmune, sclérodermie en bandes, maladie de Batten, hémophilie A acquise, polychondrite chronique atrophiante, syndrome d'Isaac (neuro-myotonie acquise), encéphalite de Rasmussen, syndrome de Morvan, syndrome de l'homme raide, anémie pernicieuse, syndrome de Vogt-Koyanagi-Harada, cirrhose biliaire primaire, hépatite auto-immune de type I, hépatite auto-immune de type II, lupus érythémateux aigu disséminé, polyarthrite rhumatoïde, polymyosite/dermatomyosite, syndrome de Sjögren, sclérodermie, vitiligo, et pelade.

17. Procédé de production d'une nanoparticule, comportant :
i) obtenir un polymère amphiphile présentant une masse molaire moyenne en nombre (Mn) de 6000 à 1000 g/mol,
ii) en option, purifier ce polymère amphiphile,
iii) faire se former des micelles de ce polymère amphiphile,
iv) et ajouter au moins un peptide comportant au moins un épitope pour lymphocyte T, afin que se forment des nanoparticules.

18. Procédé conforme à la revendication 17, dans lequel l'étape (i) est une étape de synthèse par copolymérisation par voie radicalaire, dans laquelle on utilise de préférence, en qu'amorceur de radicaux, du 2,2'-azo-bis(2-méthyl-propionitrile).
